(19) Europäisches Patentamt
European Patent Office
Office européen des brevets

(11) EP 0 547 031 B1

(12) EUROPEAN PATENT SPECIFICATION

(45) Date of publication and mention of the grant of the patent:
**23.04.1997 Bulletin 1997/17**

(51) Int. Cl.$^6$: **C07C 233/47**

(21) Application number: **93102033.3**

(22) Date of filing: **19.06.1990**

(54) **N-protected-(S)-isoserine compounds**

N-Geschützte (S)-Isoserin-Verbindungen

(S)-Isoserin-composés protégés sur l'azote

(84) Designated Contracting States:
**AT BE CH DE DK ES FR GB GR IT LI LU NL SE**

(30) Priority: **21.06.1989 US 369578**

(43) Date of publication of application:
**16.06.1993 Bulletin 1993/24**

(62) Document number(s) of the earlier application(s) in accordance with Art. 76 EPC:
**90306657.9 / 0 405 820**

(73) Proprietor: **SCHERING CORPORATION**
**Kenilworth New Jersey 07033 (US)**

(72) Inventors:
- **Tann, Chou-Hong**
  **Berkely Heights, New Jersey 07922 (US)**
- **Thiruvengadam, Tiruvettipuram Kannappan**
  **Edison, New Jersey 08820 (US)**
- **Chiu, John Sze-Hung**
  **Parsippany, New Jersey 07054 (US)**
- **Colon, Cesar**
  **Rahway, New Jersey 07065 (US)**
- **Green, Michael D.**
  **Paterson, New Jersey 07513 (US)**

(74) Representative: **Schlich, George William et al**
**Mathys & Squire**
**100 Grays Inn Road**
**London WC1X 8AL (GB)**

(56) References cited:
**FR-A- 2 441 631**

- **J.P. GREENSTEIN & M. WINITZ 'Chemistry of the Amino Acids' , JOHN WILEY AND SONS INC. , NEW YORK**
- **T.W. GREENE 'Protecting Groups in Organic Synthesis' , JOHN WILEY AND SONS , NEW YORK**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 547 031 B1

## Description

This invention relates to N-protected-(S)-isoserine compounds and to active esters thereof.

The compounds of the invention are useful in a novel process for converting gentamicin B to isepamicin, 1-N-[(S)-3-amino-2-hydroxypropionyl] gentamicin B using a novel formylating agent, 2-formylmercaptobenzothiazole. The formylating agent the process and the intermediates of the process are the subject of European patent application number 90306657.9 (EP-A-0 405 820) of which the present application is a divisional application.

More particularly, the compounds of this invention are of use in a process for converting gentamicin B to 3,6'-di-N-formylgentamicin B by using 2-formylmercaptobenzothiazole, acylation of the 1-amino group with an (S)-isoserine derivative of the invention, followed by removal of the protecting groups under conditions which result in high yields of the desired product.

Isepamicin, which has the formula

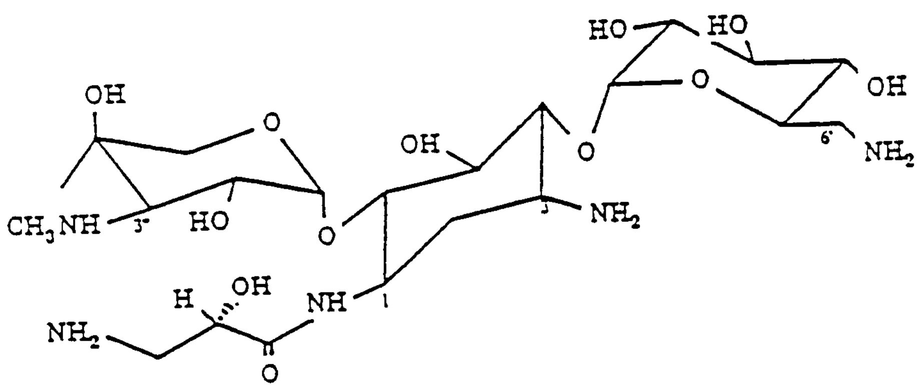

I

is a known aminoglycoside antibiotic. The preparation of this compound from gentamicin B is described in U.S. Patent No. 4,230,847. The process described in the patent involves forming 3,6'-di-N-benzyloxycarbonyl gentamicin B by reacting a cupric-nickel (II) salt complex of gentamicin B with N-benzyloxycarbonyloxy-phthalimide and then reacting the intermediate compound with N-(S-3-benzyloxycarbonylamino-2-hydroxypropionyloxy) succinimide. The benzyloxycarbonyl protecting groups were removed from the resulting material by catalytic hydrogenation over palladium-on-carbon to produce isepamicin in 60% yield from the intermediate starting material.

Tsuchiya, et al., Tetrahedron Letters No. 51, pp 4951-4954 (1979), describe a complex multistep process for the protection of the 3,3",6'-amino groups in Kanamycin A comprising zinc acetate chelation, 3,6'-N-bisbenzyloxycarbonylation, zinc removal, carbonate formation, and finally trifluoroacetylation of the 3"-amino group. The 3,3",6'-N-triblocked Kanamycin A so produced is then acylated at the free C-1 amino group using an active ester of 1-N-[(S)-4-benzyloxycarbonylamino]-2-hydroxybutyric acid. Finally the resulting product is subjected to a 2-part deprotection scheme to give Amikacin. A similar sequence is also described for the conversion of dibekacin to its 1-N-[(S)-4-amino-2-hydroxybutyryl] derivative. No reference to the use of this process for the selective acylation of other aminoglycosides was disclosed. The Tsuchiya et al. process sequence is cumbersome involving both trifluoroacetylation and benzyloxycarbonylation in the protection steps and requiring both aminolysis and hydrogenolysis in the deprotection steps. These steps render the process commercially unattractive both in the sense of operating costs and capital requirements for implementation. Furthermore, in our hands, and contrary to the implications in the Tsuchiya et al. process description, zinc acetate chelation does not invariably lead to selective 3,6'-diblockade in aminoglycosides other than Kanamycin and dibekacin. Thus, unexpectedly, the zinc acetate chelation of gentamicin B followed by acylation with formylimidazole leads primarily to 1,6'-N-diformylation and <u>not</u> 3,6'-diformylation. Again acylation of this same gentamicin B zinc acetate chelate with a different formylating agent, formylacetic mixed anhydride, gives rise, in addition to the desired 3,6'-N-diformyl-gentamicin B, to undesired levels of acetylated gentamicin B products. To underline the difficulty of prediction, formyl p-nitrobenzoic mixed anhydride proved insufficiently reactive to be useful in the formylation of gentamicin B zinc acetate chelate, whereas use of formyl p-anisic mixed anhydride afforded excellent yields of the desired 3,6'-diformyl gentamicin B, contaminated with minor amounts of anisoyl impurities.

Use of metal acetates, for example, zinc acetate and the like, give rise to small amounts of undesirable by-products, for example N-acetyl derivatives, which are difficult to remove and decrease the yield of desired product.

The formylation of aminoglycosides has been previously described by Thomas et al., Tetrahedron Letters, Vol. 21,

pp 4981-4984 (1980) in connection with the preparation of 1-N-alkylated Kanamycin antibiotics. However, Thomas et al. do not teach the value of formylation as an amine-protecting group in producing 1-N-acylated aminoglycosides. Nor is this point recognized in any other aminoglycoside literature. Instead, the literature describes the use of such as trifluoroacetyl, trichloroacetyl, and phthaloyl groups for the protection of amine groups and teaches that the aminolysis or hydrazinolysis of such groups can be carried out without materially affecting certain other N-acyl groups which may be present on the molecule. In short the selective aqueous base hydrolysis of formyl groups from 3,6'-N-formylated-1-N-acylated aminoglycosides is unprecedented.

We have now found that a novel formylating agent 2-formylmercaptobenzothiazole of formula II,

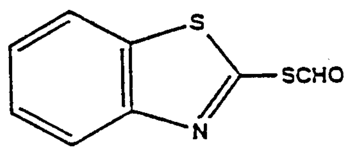

II

can selectively formylate the 3,6'-amino groups of the gentamicin B zinc chelate. Moreover, the procedure results in a high yield if one uses a different zinc salt, such as zinc pivaloate, to avoid formation of undesirable by-products. Furthermore, we have found that a 3,6'-N-diformyl gentamicin B obtained, by removing zinc from the chelate, can be selectively acylated with N-formyl-(S)-isoserine active ester only at the C-1 amino group without separate protection of the C-3" methylamino group as in the Tsuchiya et al. procedure. Finally, we have found that all formyl groups can be removed from the resulting 3,6'-N-diformyl-1-N-[N-formyl-(S)-isoserinoyl]-gentamicin B by aqueous base hydrolysis in high yield without removing the desired isoserine side chain.

This invention relates to N-protected-(S)-isoserine compounds and to active esters thereof for use in an improved multistep process for converting gentamicin B to isepamicin in high yields.

The isoserine compounds of the invention are:-

(i) N-formyl-(S)-isoserine, and
(ii) N,O-diformyl-(S)-isoserine.

The compounds of the invention are used in a process comprising the steps of:-

(a) reacting gentamicin B with a chelating agent and then with 2-formylmercaptobenzothiazole capable of selectively introducing formyl groups in gentamicin B to form 3,6'-di-N-formylgentamicin B.
(b) acylating the 1-amino group of 3,6'-di-N-formylgentamicin B with an activated N-acyl protected (S)-isoserine compound;
(c) removing all protecting groups; and
(d) isolating isepamicin.

The intermediate compound, 3,6'-di-N-formylgentamicin B, is prepared by reacting a divalent metal salt complex of gentamicin B with 2-formylmercaptobenzothiazole to introduce formyl protecting groups at the 3,6'-positions. The metal salt complex is prepared using methods disclosed in U.S. Patent No. 4,136,254 and Thomas, et al., Tetrahedron Letter, Vol. 21, 4981-4984 (1980).

The reaction scheme for preparing 3,6'-di-N-formylgentamicin B (III) is set forth below:

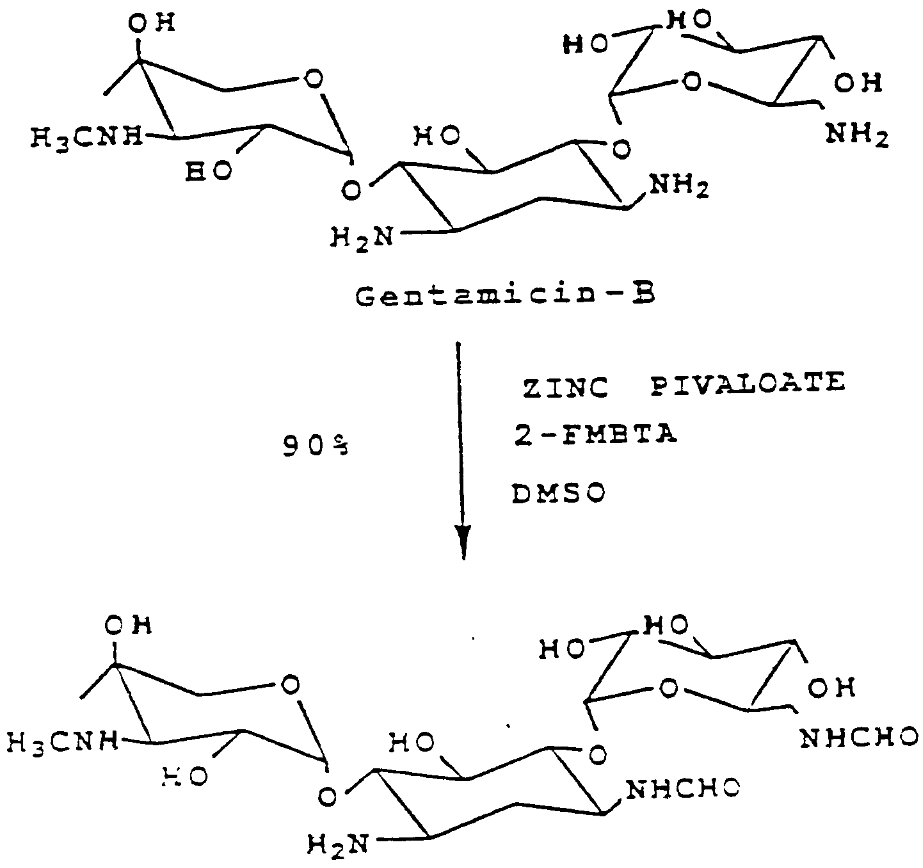


(III)

Transition metal salts useful as complexing agents in the process include such divalent salts as copper (II), nickel (II), cobalt (II), cadmium (II) and zinc (II) as well as mixtures thereof. The divalent metal salts are salts of organic acids, preferably organic acids such as formic, acetic, propionic, pivalic and benzoic acid. Preferred divalent metal salts include pivaloate salts of zinc (II) and cobalt (II). Of particular use is zinc (II) pivaloate.

The formation of the divalent salt complex of gentamicin B is carried out in an inert organic solvent. Preferred organic solvents are for example, dimethylsulfoxide, dimethylformamide, dimethylacetamide, methylene chloride, toluene, ethyl acetate and mixtures thereof.

In preparing the divalent salt complex of gentamicin B, it has been found advantageous to employ from about 1.5 - 4.5 moles of the divalent salt, for example zinc (II), per mole of gentamicin B. The preferred molar ratio of reagents is about 2.7-3.5 moles of divalent salt per mole of gentamicin B.

The divalent salt complex of gentamicin B is reacted with 2-formylmercaptobenzothiazole which introduces a formyl protecting group at both the 3 and 6'-amino groups.

The molar quantity of 2-formylmercaptobenzothiazole is usually 2-3 to 1 of the molar quantity of the divalent salt complex of gentamicin B. The preferred molar quantity is 2.5 to 1.

Formylation of the divalent salt complex of gentamicin B is carried out at a temperature of from 0°C to 40°C, preferably from 20°C to 30°C.

The formylation reaction of the divalent salt complex of gentamicin B is conveniently carried out in an organic solvent or a mixture of organic solvents. Organic solvents that can be utilized in this reaction include dipolar aprotic organic solvents, for example, dimethylsulfoxide, dimethyl formamide, dimethyl acetamide, and the like. It has also been found advantageous to employ mixtures of a dipolar aprotic organic solvent with an inert organic solvent, for example, toluene, ethyl acetate, 1,2-dimethoxyethane, tetrahydrofuran, acetonitrile, methylene chloride, and the like. A preferred mixture of solvents is dimethyl sulfoxide with either methylene chloride or ethyl acetate.

While all prior processes require the use of a precipitating agent or a procedure to remove the divalent metal salt

cation, the use of 2-formylmercaptobenzothiazole and zinc allows for an extractive removal of the zinc 2-mercaptobenzothiazole salt in the organic solvent layer.

The aqueous solution comprises 3,6'-di-N-formylgentamicin B in a yield of approximately 90-95%. The product can be isolated and purified by conventional methods such as ion exchange chromatography.

Introduction of the (S)-isoserine side-chain at the 1-amino group of 3,6'-di-N-formylgentamicin B is carried out by means of in-situ active ester formation of the N-protected-(S)-isoserine with an activating reagent in the presence of dicyclohexylcarbodiimide according to the following reaction scheme:

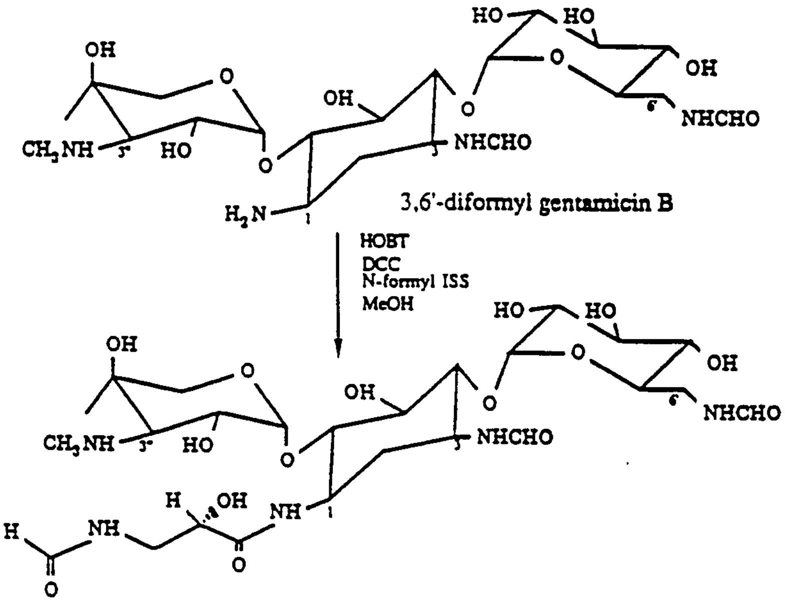


3,6'-diformyl gentamicin B

III

IV

N-Protected -(S)-isoserine compounds that are useful in the process are those wherein the amino group of -(S)-isoserine is protected with an acyl group which can be easily removed under conditions which remove formyl protecting groups and which will not affect other portions of the molecule. Acyl protecting groups which can be easily removed under mild basic conditions or with hydrazine are utilized in the process. Examples of N-acyl protecting groups which are easily removed under mild basic conditions include formyl, trichloroacetyl and trifluoroacetyl. Examples of N-acyl protecting groups which are easily removed by hydrazine include phthaloyl and succinoyl. The preferred N-acyl protecting group for the isoserine compound is the formyl group.

N-Protected isoserine compounds that are useful in the process also include N-phthaloyl-(S)-isoserine, N-trichloroacetyl-(S)-isoserine, and N-trifluoroacetyl-(S)-isoserine. The preferred N-protected isoserine compound is N-formyl-(S)-isoserine.

Active esters of N-protected-(S)-isoserine are prepared by reacting the isoserine compound with a compound such as N-hydroxybenzotriazole, N-hydroxy succinimide, imidazole, N-hydroxyphthalimide, N-hydroxy-5-norbornene-2,3-dicarboximide and the like, in the presence of a coupling agent such as dicyclohexylcarbodiimide.

The reaction of N-protected -(S)-isoserine with 3,6'-di-N-formyl gentamicin B is carried out at temperatures between 0°C and 40°C, preferably at about room temperature, in a solvent. Examples of solvents which can be employed in the process of this invention include protic organic solvents, for example alcohols, such as methanol, ethanol, propanol and the like; mixtures of water and alcohol, such as aqueous methanol, aqueous ethanol, and the like; aprotic solvents, such as dimethyl formamide, dioxane, methylene chloride. A preferred solvent is aqueous methanol.

The compound obtained by reacting N-formylisoserine with 3,6'-di-N-formyl gentamicin B is triformylisepamicin, compound IV.

The protecting groups are removed from the Compound IV by hydrolysis according to the following reaction scheme.

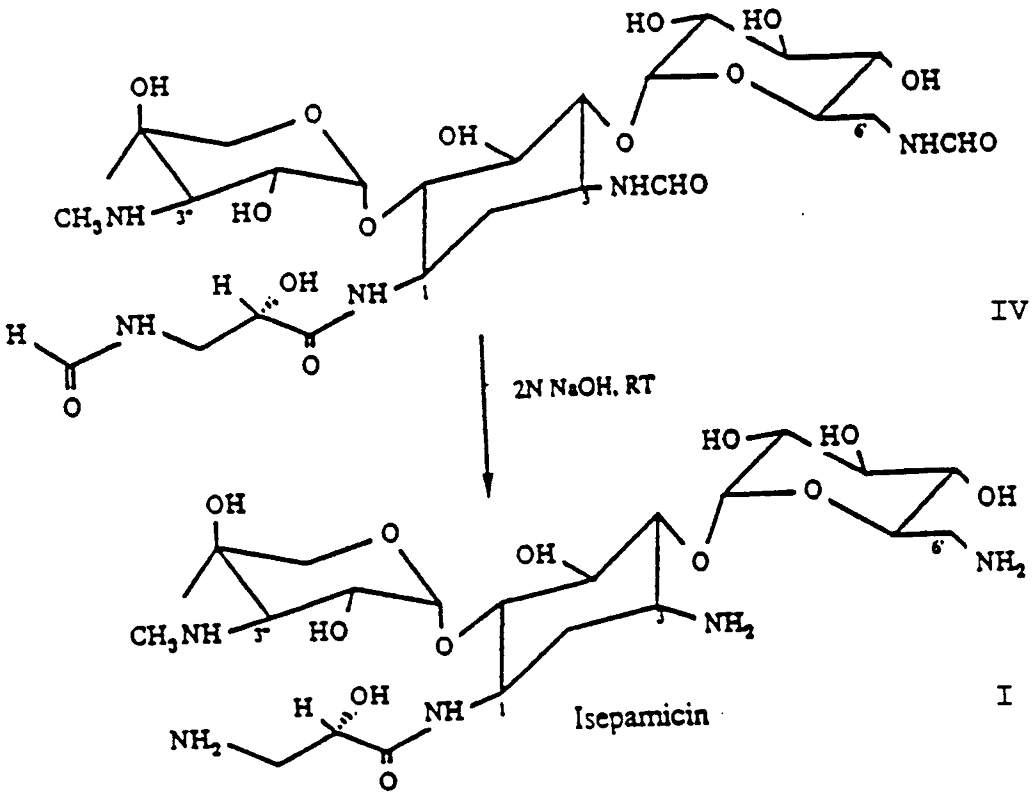


IV

I

Isepamicin

Prior to deblocking Compound IV, the solvent is removed from the reaction mixture. Although deblocking by hydrolysis is a conventional procedure the specificity for formyl group removal without removal of the isoserine side chain is unprecedented in the aminoglycoside field. It has been found that when the hydrolysis reaction is conducted by stirring overnight at room temperature, an excellent yield (88-90%) of desired product is obtained. The resulting hydrolysate is acidified to pH 6 with acid and isepamicin is obtained by isolation.

In the example HPLC means High Performance Liquid Chromatography; Amberlite IRC-50 is a weak cation ion exchange resin available from Rohm and Haas Company.

Example 1

Preparation of N,O-Diformyl-(S)-Isoserine

To a one liter round bottom flask containing 50 g of (S)-isoserine (.476 mole) and 62.5 ml of formic acid was added in 30 min. a freshly prepared acetic formic anhydride solution * (5 eq.) at 0-5°C. After examining the completion of reaction by H$^1$-NMR (approx. 2 hrs), the mixture was concentrated under vacuum at 40°C to half of the original volume. 250 ml of isopropanol was added slowly with simultaneous cooling to effect crystallization. The slurry was stirred at 0°C for one hour. The product, N,O-diformyl-(S)-isoserine, was filtered and washed with isopropanol. This afforded 64 g of N,O-diformyl-(S)-isoserine; 84% yield; m.p. 139.5°-141.5°; $[a]_D^{20}$ : -38° (1%, MeOH).

$^1$H-NMR ($D_2O$) w 3.8 (dd, 1H, J=14.6, 4.4 Hz), 3.91 (dd, 1H, J=14.6, 5.5 Hz), 5.38 (dd, 1H, J=5.5, 4.4 Hz), 8.15 (s, 1H), 8.27 (s, 1H).

* Acetic formic anhydride was prepared by adding acetyl chloride to 1.2 eq. of sodium formate (anhydrous, micronized) in anhydrous acetonitrile (the concentration of sodium formate/$CH_3CN$ can be as high as 50%) at 0-5°C. The reaction takes 2 hours to complete. The precipitate was filtered, the filtrate was used as is in the above reaction. Some carbon monoxide is evolved from this mixture, depending on the temperature. Reasonable stability was observed at 0°, for one month.

Example 2

Preparation of N-Phthaloyl-(S)-Isoserine

To a stirred suspension of 15.75g (150 mmole) of (S)-isoserine and 22.2g (150 mmole) of phthalic anhydride in 600 ml of toluene:dimethylformamide (3:1), was added 2.1 ml (15 mole) of triethylamine. The suspension was heated to reflux and the water generated was removed using a Dean-Stark condenser. No additional water separated after two (2) hours at reflux. The solvent was evaporated to a final volume of approximately 100 ml. The reaction mixture was cooled, diluted with ice-water and acidified with 2N hydrochloric acid to afford a precipitate. The product was filtered, washed with ice-water and dried under vacuum to yield 30.4g (86%) of N-phthaloyl-(S)-isoserine; m.p. 227-228°C; $[a]_D^{20}$ : +10 (1%, DMF).
$^1$H-NMR (DMSO-$d_6$) w 3.76 (dd, 1H, J=13.46, 7.69 Hz), 3.84 (dd, 1H, J = 13.46, 5.77 Hz), 4.3 (dd, 1H, J=7.69, 5.77 Hz), 7.77-7.89 (m, 4H).

Example 3

Preparation of N-trifluoroacetyl-S-isoserine

To a stirred solution of sodium methoxide in methanol, 11 ml (1 eq., 24.8% w/w solution) was added 5 g of (S)-isoserine. The mixture was stirred at room temperature for 15 minutes until a homogeneous solution was obtained. Ethyltrifluoroacetate, 7 ml (1.25 eq.) was added. The mixture was stirred for 30 minutes after the addition. The completeness of the reaction was monitored by $^1$H-NMR. The mixture was concentrated under reduced pressure to as low a volume as possible. To the residue 50 ml ethylacetate was added. The mixture was cooled to 0-5°C, 25 ml of 2N HCl (1 eq.) was added, followed by 5 g of solid sodium chloride. The organic layer was separated. The aqueous layer was reextracted with 50 ml of ethylacetate. The combined organic extracts were dried (over 5 g of anhydrous magnesium sulfate), filtered and concentrated under reduced pressure to 20 ml. To it 50 ml heptane was added with stirring in an ice-bath for 30 minutes. The product was filtered and dried to yield 8.86 g (93%) of N-trifluoroacetyl-(S)-isoserine; m.p. 142-143°C; $[a]_D^{20}$ : +12.4 (1%, $H_2O$);
$^1$H-NMR ($D_2O$) w 3.78 (d, 2H, J=5.48 Hz), 4.53 (t, 1H, J=5.48 Hz).

Example 4

Preparation of Isepamicin

The following methods illustrate the preparation of Isepamicin.

Method A:

A stock solution of N-formyl-(S)-isoserine was prepared by stirring 20 g (124.2 mmoles) of N,O-diformyl-(S)-isoserine in a mixture of methanol (85 ml) and pyridine (15 ml, 1.5 equiv.) at room temperature for 14-16 hrs. The completion of the reaction was judged by $^1$HNMR.

In a separate flask, 20 g of aq. concentrate (4.424 g active, 8.2 mole) of 3,6'-diformyl gentamicin B and 1.26 g (8.26 mmole) of 1-N-hydroxybenzotriazole monohydrate were dissolved in 40 ml of methanol. To the stirred mixture the above N-formyl-(S)-isoserine solution in methanol (22.2 ml 24.4 mmole, 3 equiv.) and a solution of dicyclohexyl carbodiimide (5g, 24.3 mole, 3 equiv.) in 20 ml of methanol were added simultaneously over a period of 40 min. The mixture was stirred for 15 mins. after the addition was complete. The progress of the reaction was monitored by either HPLC or by TLC. The solvents were then removed under reduced pressure, and the product, triformylisepamicin, was hydrolysed by stirring at room temperature for 16 hrs. with 90 ml of 2N NaOH. The reaction mixture was neutralised to pH=6 with acid, filtered and the filtrate was diluted to a precise volume of 1000 ml. External standard HPLC assay of this solution indicated an 89% yield of isepamicin (4.17 g, 7.3 mole).

Method B:

A solution was prepared by dissolving 1.156g (96.6% pure, 2.07 mmole) of 3,6'-di-N-formylgentamicin B, 800 mg (1.7 eq.) of N-phthaloylisoserine and 365 mg (1.2 eq.) of N-hydroxybenztriazole monohydrate in 40 ml of methanol. To this solution was added 700 mg (1.7 eq.) of dicyclohexylcarbodiimide. The reaction was stirred at room temperature for one hour and 160 mg of N-phthaloyl-(S)-isoserine and 140 mg of dicyclohexylcarbodiimide was added and the reaction was allowed to stir at room temperature for approximately three (3) hours. The progress of the reaction was monitored by TLC. The solvent was removed by evaporation and the residue taken up in 50 ml ethanol and 5 ml water. The protecting groups were removed by treating the resulting mixture with 6.0 ml (85%) hydrazine hydrate. The reaction was heated at 85-90°C under nitrogen for 14 hours. External standard HPLC assay of the reaction indicated a yield of 89% (1.05 g, 1.85 mmole) isepamicin.

Method C:

To 48.9 g of aq. concentrate (8.45 g active, 15.7 mmole) of 3,6'-diformylgentamicin B, 2.4 g (15.7 mmole) of 1-N-hydroxybenzotriazole monohydrate was added followed by 80 ml of methanol. To the stirred mixture, 9.5 g (47.3 mmole, 3 equiv.) of N-trifluoroacetyl-(S)-isoserine in 40 ml of methanol and 9.7 g (47.1 mmole, 3 equiv.) of dicyclohexyl carbodiimide in 40 ml of methanol were added simultaneously over a period of 40 min. The mixture was stirred for 15 min. after the addition was complete. The progress of the reaction was monitored by either HPLC or TLC. The solvents were then removed under reduced pressure, and the product was hydrolysed by stirring with 170 ml of 2N NaOH at room temperature for 16 hrs. The reaction mixture was neutralised to pH=6 with acid, filtered and the filtrate was diluted to a precise volume of 1000 ml. External standard HPLC assay of the solution indicated an 88% yield of isepamicin (7.84 g, 13.8 mmole).

## Claims

1. N,O-diformyl-(S)-isoserine.

2. N-formyl-(S)-isoserine.

3. A method of making an active ester of an N-protected-(S)-isoserine compound, comprising reacting an isoserine compound according to Claim 1 or Claim 2 with a compound selected from N-hydroxybenzotriazole, N-hydroxysuccinimide, imidazole, N-hydroxyphthalimide and N-hydroxy-5-norbornene-2,3-dicarboximide in the presence of a coupling agent.

4. A method according to Claim 3 in which the coupling agent is dicyclohexyl-carbodiimide.

5. A method according to Claim 3 or 4 in which the isoserine compound is reacted with N-hydroxy-benzotriazole.

## Patentansprüche

1. N,O-Diformyl-(S)-isoserin.

2. N-Formyl-(S)-isoserin.

3. Verfahren zur Herstellung eines aktiven Esters einer N-geschützten (S)-Isoserinverbindung, umfassend das Umsetzen einer Isoserinverbindung gemäß Anspruch 1 oder Anspruch 2 mit einer Verbindung, die aus N-Hydroxybenzotriazol, N-Hydroxysuccinimid, Imidazol, N-Hydroxyphthalimid und N-Hydroxy-5-norbornen-2,3-dicarboximid ausgewählt ist, in Gegenwart eines Kopplungsmittels.

4. Verfahren gemäß Anspruch 3, wobei es sich bei dem Kopplungsmittel um Dicyclohexylcarbodiimid handelt.

5. Verfahren gemäß Anspruch 3 oder 4, wobei die Isoserinverbindung mit N-Hydroxybenzotriazol umgesetzt wird.

## Revendications

1. N,O-diformyl-(S)-isosérine.

2. N-formyl-(S)-isosérine.

3. Méthode de fabrication d'un ester actif d'un composé de (S)-isosérine N-protégée, comprenant la réaction d'un composé isosérine selon la revendication 1 ou la revendication 2 avec un composé choisi parmi le N-hydroxybenzotriazole, le N-hydroxysuccinimide, l'imidazole, le N-hydroxyphthalimide et le N-hydroxy-5-norbornène-2,3-dicarboximide en présence d'un agent de couplage.

4. Méthode selon la revendication 3, dans laquelle l'agent de couplage est le dicyclohexyl-carbodiimide.

5. Méthode selon la revendication 3 ou 4 dans laquelle le composé isosérine est mis à réagir avec du N-hydroxybenzotriazole.